# EUROPEAN PATENT SPECIFICATION

(11) **EP 2 076 139 B1**
(45) Date of publication and mention of the grant of the patent: **07.12.2011**
(21) Application number: 07719227.6
(22) Date of filing: 14.03.2007
(51) Int. Cl.: A23C 9/127, A23L 1/30, A61K 35/74, C12R 1/225

(54) **PROBIOTIC STRAIN LACTOBACILLUS DELBRUECKII SUBSP . BULGARICUS**
PROBIOTISCHER STAMM VON LACTOBACILLUS DELBRUECKII SUBSP BULGARICUS
SOUCHE PROBIOTIQUE DU LACTOBACILLE DELBRUECKII SUBSP. BULGARICUS

(30) Priority: 09.10.2006 BG 10969906
(43) Date of publication of application: 08.07.2009
(73) Proprietor: Baltadjieva, Maria, 4002 Plovdiv (BG); De Las Heras, , Jose Miguel, 28760 Madrid (ES)
(72) Inventor: Baltadjieva, Maria, 4002 Plovdiv (BG); De Las Heras, , Jose Miguel, 28760 Madrid (ES)
(74) Representative: Stefanova, Stanislava Hristova
(86) International application number: PCT/BG2007/000004
(87) International publication number: WO 2008/043161

(56) References cited:
- FR-A1- 2 411 001
- US-A1- 2004 047 849
- DATABASE EPODOC EUROPEAN PATENT OFFICE, THE HAGUE, NL; Patent BG108615, only database entry available, 31 October 2005 (2005-10-31), LAKTINA OOD (BG): "Synbiotic" XP002441206
- GUARNER FRANCISCO ET AL: "Should yoghurt cultures be considered probiotic?" BRITISH JOURNAL OF NUTRITION, vol. 93, no. 6, June 2005 (2005-06), pages 783-786, XP002441358 ISSN: 0007-1145
- PENNER ET AL: "Probiotics and nutraceuticals: non-medicinal treatments of gastrointestinal diseases" CURRENT OPINION IN PHARMACOLOGY, ELSEVIER SCIENCE PUBLISHERS,, NL, vol. 5, no. 6, December 2005 (2005-12), pages 596-603, XP005134770 ISSN: 1471-4892
- SCHEINBACH SAUL: "Probiotics: Functionality and commercial status" BIOTECHNOLOGY ADVANCES, vol. 16, no. 3, May 1998 (1998-05), pages 581-608, XP009086233 ISSN: 0734-9750 cited in the application

## Description

### Field of invention

The invention relates to strain *Lactobacillus delbrueckii subsp. bulgaricus* c with a broad spectrum of use, more precisely to the strain *Lactobacillus delbrueckii subsp. bulgaricus,* registered in the National Bank for Industrial Microorganisms and Cell Cultures (NBIMCC) under No 8481 for preparation of a products of pharmaceutical and alimentary type as a prophylactic, therapeutic and medicinal means with a wholesome effect to the human organism.

### Background of the invention

Investigations on the inherent of the human digestive system microorganisms- *Lactobacillus, Streptococcus, Bifidobacterium or Enterococcus,* showed their wholesome effect to the lactose intolerance, sera cholesterol, immune system, as to their ability to control the infections through sustaining a healthful balance of the intestine microflora (1). At the same time in many cases there are absent of statistically reliable data for the wholesome effect of the commercial microorganisms. The main technical problem remains the selection of such strains lactic microorganisms, which could be used for a preparation of products with appropriate organoleptic qualities and clearly expressed dietetic, prophylactic and therapeutic characteristics.

It is known from patent Nos. UA63201, UA63202 and UA64163 that if strain *Lactobacillus delbrueckii subsp. bulgaricus* 9702 is cultivated under specific condition it produce a biologically active substance with a hepatoprotective, gastroenterological and immune modulating properties. But concrete clinical experimental data, supporting such affirmation are not showed.

It is also known from patents BG63961, BG63962 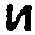 BG63963 that special strains *Lactobacillus delbrueckii subsp. bulgaricus* in combination with special strains of genus *Streptococcus thermophilus* in special proportions, are used as a starter cultures for a Bulgarian yogurt and other fermented milk products.

Different possible applications of strains *Lactobacillus delbrueckii subsp. bulgaricus* in a combination with different nature products are known from patent BG61476. It describes the possibility of combination the biologically active substance, produced by a culture liquor of strain *Lactobacillus delbrueckii subsp. bulgaricus* with nature products as metarine, hydrolyzed lactic casein, apple pectin, balanced vitamin complex, fructose, menthol, sweeteners and mineral salts for preparation of food additive.

All above described patents do not show data illustrating the steadiness of microorganisms during the different technological processes; the direction of applicability of the known strains is limited to the area of fermented milk products; the cultures are tested only under lactic base; data for their growing and development during the processes of rennet- and rennet- acid fermentation are not shown, which is limiting their use only to the diary products; there are not data concerning the preservation of activity under different conditions and mediums; the scope of their action is limited to a short time; the conditions in which their maximum of activity is revealed are not cleared.

### Description of the invention

The invention relates to strain *Lactobacillus delbrueckii subsp. bulgaricus,* registered in NBIMCC under No. 8481 with a broad spectrum of use, more precisely for preparation of products of pharmaceutical and alimentary type as a prophylactic, therapeutic and medicinal means with a wholesome effect to the human organism. The strain is isolated in the district *Stara planina* from a fresh cheese made of cow milk, fermented five days, with a subsequent selection.

Morphologically the strain is characterized with a rod- like cells with a rounded ends, disposed individually, in a group of two or as a chains. It forms white, compact colony, do not forms spores. The strain is Gram (+), homofermentative, facultative anaerobe. It has a broad temperature range of development- from 35°C to 45°C and is stable during 11 months under temperature 2°C; the strain is stable, too, when is lyophilized, which simultaneously gives the advantage of production standardized product and of providing high level concentration of live cells in the final product.

The strain is catalase negative and removes the fence of glucose to two molecules lactic acid- acetilmetilcarbinole, acetoine and diacetile in ration 2:1:3; it ferments the saccharose and fructose to 60%, lactose to over 65%, mannose- to 70%. A typical feature for the strain *Lactobacillus delbrueckii subsp. bulgaricus,* NBIMCC No8481 is its high level post acid forming ability under comparatively high cell's activity under low values of pH (<4,0); it produces a broad range of metabolite products, lactic acids and enzymes with clearly marked proteolytic properties; it grows very well in a medium of milk with a rennet- and rennet- acid coagulation and the quantity of the presented calcium dichloride has no influence on it.

The strain is stable on the antibiotics as follows: *Penicylin, Gentamicin, Kanamycin, Streptomycin, Vancomycin, Erythromycin, Chloramphenicol, Quinupristin-Dalfopristin, Fusidic acid, Tetracyclin, Moxifloxacin, Linezolid, Ampicillin, Teicoplanin* and others, which is illustrated with a data of the applied Table 1.

**Table 1**

| Pen | Amp | Gm | Km | Sm | Fus | Tet | Van | Tei | Er | Clor | Lin | Moxi | Q/ D | Ac Desoxi 2% | pH=1 |
|---|---|---|---|---|---|---|---|---|---|---|---|---|---|---|---|
| <0,0 6 | <0,0 6 | 32 | 16 | >64 | >64 | 1 | >10 24 | >32 | 1 | 16 | <0,5 | 8 | 1 | grows | + |

Shortenings: Pen- penicilin; Amp- ampicilin; Gm- gentamicin; Km- kanamycin; Sm- streptomycin; Fus- fusidic acid; Tet- tetracyclin; Er- erythromycin; Clor-chloramphenicol; Lin- linezolid; Moxi- moxifloxacin; Q/D- quinupristin/dalfopristin; Van- vancomycin; Tei- teicoplanin;

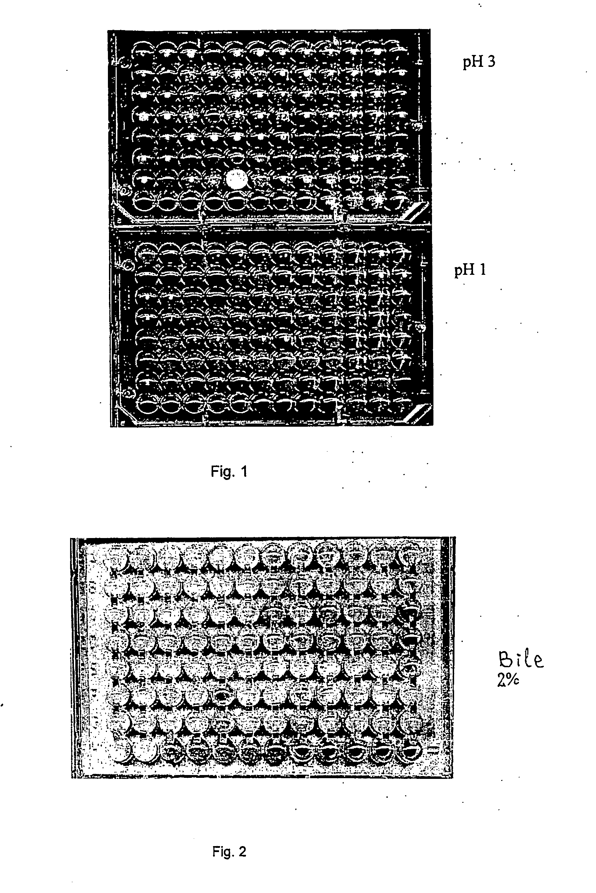

A quality analysis with a conventional PCR and DGGE tests towards a lactoacid microflore of fecal samples of voluntaries which were given the strain show positive growing to the specific level of *Lactobacillus delbrueckii subsp. bulgaricus NBIMCC Nº 8481*, coinciding with the positive control sample, received from yoghurt, fermented with the strain according to the present invention. The conclusion is that the strain shows a high level of surviving in the intestine (Fig. 1).

The strain demonstrates, too, a survival under pH greater than 2, which is an indication for surviving in the acid stomach medium. Experiments under different concentrations of bile acids show very good survival of the strain under concentration of 2%. (Fig. 2)

Quantitative PCR investigations regarding the inhibiting influence of strain *Lactobacillus delbrueckii subsp. bulgaricus NBIMCC Nº 8481* versus pathogen microorganisms of the genus *Bacteroides, Prevotella, Porphyromonas and Clostridium* show a significant lowering (till 58%) of their numbers in the voluntary's fecal after taking the strain according to the invention. For the genus *Bacterium vulgaris* the lowering of the bacterial numbers come to 41 %.

The experimental determine of the numbers of shortchain organic acids in the fecal probes of voluntaries which have been given the strain show also lowering of the contents of acids as acid, propionic and butyric, which have the effect to stimulate the intestine epithelium grow.

The cells of the strain show very high resistance to organic additives in the lactic medium as sugars, fruits, pectin, tannin, vitamins, colorants and fragrances.

The above described qualities give the opportunity of broad spectrum of use of the strain, according to the present invention, as a monoculture and as a mixed culture with other microorganisms for preparation of products of pharmaceutical and alimentary type as a prophylactic, therapeutic and medicinal means with a wholesome effect to the gastrointestinal tract and other organs and systems of the human organism. The products of pharmaceutical type in their entity of anticholesterol and geriatric preparations, preparations against mental and physical over fatigue are prepared as oral pharmaceuticals- tablets, capsules, liquid suspensions, dry oral supplements and some more containing a bacterial charge of alive cells of *Lactobacillus delbrueckii subsp. bulgaricus NBIMCC Nº 8481* between 2 x 10⁹ to 2 x 10¹¹.

The same quantity of live cells, according to the present invention, can be included as an active component in the process of preparation various kind of alimentary products as fermented milk products, different kind cheeses- fresh, semisolid and solid, protein concentrates, symbiotic products, children's food, dry lactic products and others.

Simultaneously this strain favorable influences the organoleptic properties of the final products, adding a specific aroma due to its active metabolism and as a result of lactose degradation; its feature to produce enzymes with a proteolytic properties leads to the production of a fermented lactic beverages with a homogeneous and viscose consistence and with a low syneress and to the improvement of cheese's consistence, more precisely that of the fresh and semisolid cheeses.

The property to produce bacteriocines gives the opportunity of using the strain as a products of a pharmaceutical or alimentary type as agents against pathogenic organisms in different inflammatory processes; its stability to antibiotics allows it's simultaneously application or its use as a finalizing the health process factor.

### Briefly Description of the Figures

Fig. 1- shows the survival of strain *Lactobacillus delbrueckii subsp. bulgaricus NBIMCC No 8481* in medium with pH 2,0- 3,0 during 180 min.
Fig. 2 shows the survival of strain *Lactobacillus delbrueckii subsp. bulgaricus NBIMCC No* 8481 in the presence of bile acid in a concentration 2%.

The following Examples illustrate the present invention without limiting its spirit and scope of protection.

### Example 1: Preparation of biologically pure culture of the strain Lactobacillus delbrueckii subsp. bulgaricus NBIMCC No 8481

The strain is isolated in the district *Stara planina* from a fresh cheese made of cow milk, fermented five days, with a subsequent selection. The bacterial culture is produced first in laboratory as follows: 1 g of the product is suspended in a 9 ml sterile physiological solution after that repeated dilutions are made and of dilution 10⁷ a sow is made onto a MRS- agar medium for lactobacillus. The cultivation is carry out under temperature 44-45°C during 48 hours. The strain is selected from the final single colonies. The selected strain is sowed again in 10 ml sterile skimmed milk till coagulation pH 4,8 for 6 hours. From this culture, named "primary culture" of the strain a subcultures are made with the aim to increase the number of cells of the pure strain and to prepare a "mother cultures", which later to be used as an inoculant during the industrial preparation of bacterial culture of strain *Lactobacillus delbrueckii subsp. bulgaricus NBIMCC No 8481.*

### Example 2: Use of strain Lactobacillus delbrueckii subsp. bulgaricus NBIMCC No 8481 for preparation a synbiotic product

The product is suggested in two variants- in a liquid or in a lyophilized form. For its preparation, 100 l milk is skimmed to 0,05% and is sterilized on two steps: under temperature 105- 110°C for 30 minutes and under temperature 119- 125°C for 30 minutes, after which it is cooled to temperature 45- 47°C. To the cooled milk a 2 kg preliminary prepared culture is added, composed of specially selected strain *Lactobacillus delbrueckii subsp. bulgaricus NBIMCC No 8481* in a combination with *Streptococcus thermophilus NBIMCC No 8357*- two parts, *Lactobacillus acidophilus NBIMCC No 8358*- one part and *Lactobacilus bifidus-* three parts. The inoculated milk is fermented under temperature regimes as follows: stage I- under temperature 45- 47°C for 60 minutes; stage II- under 40- 41 °C for 30 minutes and stage III- under 37- 38°C until pH 5,3 is reached for production of a liquid symbiotic product. After that to the product are added syrups made of fruits and sugar until reaching a dry substance 60%, it is cooled quickly to 20°C for 20 minutes, distributes in a packages of 30 ml and 50 ml and it is kept under temperature 3-4°C.

For preparation of dry synbiotic product, the coagulated milk with pH 4,9- 5,0 is submitted on two steps cooling: step I- to 25°C for a 20- 25 minutes and stage II- to 6-7°C for a 15 to 20 minutes, after which it is kept during 24 hours under temperature -38°C to 40°C and it is drying using the method of lyophilisation until the product reaches liquor contents of 4- 6%. It is packed in packages of 5, 10 and 20 g.

The prepared under above described technology product is a symbiotic (pro- and pre- biotic), which includes complete milk protein components, microelements, mineral salts and vitamins. After all, the product has a guarantied long lasting stability- under temperature+3°C to +4°C it is 6 months and under temperature from - 8°C to -10°C the stability reaches 18 months.

The bacterial charge of alive cells of *Lactobacillus delbrueckii subsp. bulgaricus NBIMCC Nº 8481* is between 2 x 10⁶ to 2 x 10¹⁰, estimated according to microbiological methods of bacterial count known to a man skilled in the art, like for instance those reported in *Ison A. P. and Matthew G. B.* (2).

### Example 3: Use of strain Lactobacillus delbrueckii subsp. bulgaricus NBIMCC No 8481 for preparation of a starter culture for a fermented milk probiotic products

For preparation starter culture, two groups of symbiotic cultures are created as follows: symbiotic culture of strain *Lactobacillus delbrueckii subsp. bulgaricus NBIMCC No 8481 and strain Streptococcus thermophilus NBIMCC No 8357 in a ratio* 1:2 are repeatedly sow during 2,6 months until the symbiosis is stabilized. After that, two parts of the symbiotic pair is mixed with one part of culture of Lactobacillus acidophilus NBIMCC No 8358 and in view to stabilizing culture 16- fold repeated sows in a skimmed cow milk under temperature 43°C until coagulation for time limit of 3 hours. As a result a product with a bacterial consistence on the average of 2,8.10⁹ Cfu/g is produced. In view to raise stability and transportability of the product, a lyophilisation is conducted as follows: the condition of active cells is preliminary stabilized in a protective medium, consisting of skimmed milk, saccharose, glucose, lactose and maltose in a preliminary cooling firstly under temperature of 25°C during 20- 25 min and after that- under temperature of 6-7°C during 15- 20 min, than the product is kept for 24 hours under temperature of -38°C to -40°C and is lyophilized till water consistence of 4-6%. Kept under temperature of 4-6°C, the starter cultures preserve their activity during 8 months and under temperature -10°C to -18°C, their activity is preserved for more than 18 months.

Except for the preparation of a traditional fermented milk products- yoghurt and cheese, the starter culture could be used directly in a dry form- in bulk or as a tablet form, for sportsmen's needs, for diabetics, as an immunostimulant, for recovering ill people and many other applications. The dry product show good dermatological effect and finds successful application in cosmetics.

### Example 4: Use of strain Lactobacillus delbrueckii subsp. bulgaricus NBIMCC No 8481 for the preparation of a fermented milk probiotic product

For preparation of a fermented milk probiotic product, 1000 I fresh milk-caw's, buffalo's, goat's or a mix of them with acidity 17°T, qualified according to the standards, is warmed to the temperature 35°T and is purified through a separator. The milk is normalized with a skimmed milk to a fat contents 2,3% and 3,6%, is warmed to a temperature 60°C and is homogenized under pressure 18 MPa, is pasteurized to a temperature 97° for a 30 minutes and is cooled to 44°C. After that pectin and tannin, dissolved in a water in advance are added in quantity respectively 0,25% and 0,015%. Treated in a such way milk is fermented with a 2% starter culture, composed of *Lactobacillus delbrueckii subsp. bulgaricus,* NBIMCC No 8481 *and Streptococcus thermophilus NBIMCC No 8357* in a proportion of 1:2 under 45°C until obtaining a visible coagulation. The coagulated milk is leaved for a 40 minutes to be cooled to 20°C and the final product is kept under 4°C until its distribution on the market.

The final product, prepared as it is described in the given Example has the typical organoleptic and biological characteristics of the traditional Bulgarian yoghurt, but contains substances with protective properties towards heavy metals. This makes it very appropriate for consummation by a people working in environment with an increased presence of heavy metals.

### Example 5: Use of strain Lactobacillus delbrueckii subsp. bulgaricus NBIMCC No 8481 for the preparation of a biologically active product

Biologically active fermented milk product, according to the present Example includes whey, which is pasteurized in advance and fermented with a starter culture consisting of strains *Lactobacterium delbrueckii ssp. bulgaricus* NBIMCC No.*8481*, *Streptococcus thermophilus NBIMCC No.8357* and lactosofermeting jeasts of the type *Saccharomyces lactis* in a ratio 4:2:1; extracts of the medicinal herbs *Teucrium chamaedrae, Flores chamomillae, Herba millefolium, Hypericum perforatum* and *Glycyrrhiza glabra* in a ratio 2:1:1:1:3; saccharose and a stabilizer. The final mix is thickening to dryness 45% and the product is packed.

The product contains all whey's components and is reached with the valuable metabolites of vital cells of the strain *Lactobacillus delbrueckii subsp. bulgaricus NBIMCC No. 8481*; the microflore contained in it allows to be well assimilated by children, adults and old people, as by ill people, whose enzyme system in the digestive tract is breached due to the absence of enzyme lactase; the substances contained in the medical herbs extract- tannin, azolein and others increase the stomach secretion, improve the digestion and prove the medication of gastrointestinal illnesses. The product could combine favorable fruit juices and fruit beverages. It's specific amino acid, protein and mineral composition gives it qualities of food radio protector.

### Example 6: Use of strain Lactobacillus delbrueckii subsp. bulgaricus NBIMCC No 8481 as a medicine with a wholesome effect to the gastrointestinal dislikes of children and adults

1000 l cow milk- skimmed to 0.5% is treated thermally on 105°C- 110°C, is cooled to 43°C, 1% fructose and 0,3% calcium salts, vitamin A, D and E, flavoring agents and colorants are added, after that the mixture is fermented with a starter culture, including 2,5% strain *Lactobacillus delbrueckii subsp. bulgaricus NBIMCC No. 8481* combined with a strain *Streptococcus thermophillus NBIMCC No.8357* in ratio 2:1.

The resulted product has a tender consistence, like a cream with a typical taste of the fermented milk products, but sweeter and with a specific aroma due to the presence of diacetyl after glucose degradation; the microscope picture show a field rich of rods with a typical morphology, preserving their vitality more than 30 days under temperature from 3°C to 6°C.

The final product is suited for a children, adults and ill people with a lactase deficiency.

### Example 7: Use of strain Lactobacillus delbrueckii subsp. bulgaricus NBIMCC No 8481 for preparation of probiotic cheese

For the probiotic cheese preparation, 100 l milk is heated to a temperature 45°C after that is purified and normalized to a ratio casein: fats from 0.65 to 0.68, is pasteurized 20 minutes on a temperature 70-80°C and is cooled to 30- 35°C. The acidity is raised to 22-23 °T through 0.3- 0.5% liquid starter culture, represented of two groups of microorganisms in a ratio 1:2. The first group is composed of strain *Lactobacillus delbrueckii subsp. bulgaricus NBIMCC No .* 8481 - *and strain Streptococcus thermophilus NBIMCC No.8357* in ratio 1:3 and the second group includes strain *Streptococcus lactis NBIMCC No. 8482 and strain Lactobacillus casei* in ratio 1:2.

To the milk, treated as above, are added 30 ml 50% solution of calcium dichloride and rennet in a quantity, sufficiently to achieve the coagulation for 8 to 11 minutes. The mixture of whey and cheese grains is heated from 37 °C to 40°C during 40 to 50 minutes. The cheese grains, separated from the whey are poured in pans for squeezing and forming. The further treatment of as received pre- cheese is made on two steps for 60 minutes using well known techniques. The final product is ripening under temperature 12- 14°C during 20 to 25 days, after which is packed and kept under temperature 4 to 6 °C until its realization on the market.

### Literature

1. Shenbach S., Probiotics: Functionality and commercial status, Biotechnology Advanced, Vol. 16, No. 3, pp. 581- 608, 1998
2. Ison A. P. and Matthew G. B., Applied Microbial Physiology a practical approach, edd. Rhodes P. M. and Stanbury P. F., IRL Press Oxford, 103-130,1997.

## Claims

1. Strain *Lactobacillus delbrueckii subsp. bulgaricus* registered in the National Bank of Industrial Microorganisms and Cell Cultures (NBIMCC) under No. 8481.

2. Use of a biologically pure culture according to claim 1 for preparation of products of pharmaceutical and alimentary type as a prophylactic, therapeutic and medicinal means with a wholesome effect to the human organism.

3. Products of pharmaceutical and alimentary type of biologically pure culture of the strain of claim 1, **characterized in that** they include 2 x 10⁹ to 2 x 10¹¹ Cfu/g alive cells of biologically pure culture of strain *Lactobacillus delbrueckii subsp. bulgaricus,* registered in NBIMCC under No. 8481.

## Patentansprüche

1. Der Stamm *Lactobacillus delbrueckii subsp. bulgaricus,* registriert in der Nationalen Bank für industrielle Mikroorganismen und Zellkulturen (NBIMZK) unter No. 8481.

2. Anwendung einer biologisch reinen Kultur nach Anspruch 1 für Erhaltung von Produkten pharmazeutischer und lebensmittelartigen Typen als prophylaktischen, therapeutischen und Heilungsmittel mit vorteilhaftem Einfluß auf dem menschlichen Organismus.

3. Produkte pharmazeutischer und lebensmittelartiger Typen einer biologisch reinen Kultur nach Anspruch 1, **gekennzeichnet dadurch, dass** sie von 2x10⁹ bis 2x10¹¹ Cfu/g lebendige Zellen der biologisch reinen Kultur des Stamms *Lactobacillus delbrueckii subsp. bulgaricus,* registriert in NBIMZK unter No. 8481, enthalten.

## Revendications

1. La souche *Lactobacillus delbrueckii subsp. bulgaricus,* dont le numéro d'enregistrement à la Collection nationale de microorganismes industrielles et de cultures cellulaires (CNMICC) est le Nº 8481.

2. l'utilisation d'une culture biologique selon la revendication 1 pour l'obtention de produits de nature pharmaceutique ou alimentaire, considérés comme moyens de prévention, de thérapie, de cure ayant des effets bénéfiques sur l'organisme humain.

3. Des produits de nature pharmaceutique et alimentaire, issus d'une culture biologique de la souche selon la revendication 1, **caractérisés par le fait qu'**ils comprennent de 2x10⁹ à 2x10¹¹ Cfu/g de cellules vivantes d'une culture biologique de la souche *Lactobacillus delbrueckii subsp. bulgaricus,* dont le numéro d'enregistrement à la CNMICC est le Nº 8481.
